# EUROPEAN PATENT SPECIFICATION

(11) **EP 3 330 418 B1**
(45) Date of publication and mention of the grant of the patent: **26.06.2019**
(21) Application number: 16201797.4
(22) Date of filing: 01.12.2016
(51) Int. Cl.: D03J 1/14, D03J 1/18, B65H 69/04

(54) **YARN SEPARATING MODULE WITH A CAPACITIVE SENSOR DEVICE**
GARNTRENNMODUL MIT EINER KAPAZITIVEN SENSORVORRICHTUNG
MODULE DE SÉPARATION DE FILS AVEC UN DISPOSITIF CAPTEUR CAPACITIF

(43) Date of publication of application: 06.06.2018
(73) Proprietor: Stäubli Sargans AG, 7320 Sargans (CH)
(72) Inventor: BÜSCH, Gregory, 9475 Sevelen (CH); WIRTH, Matthias, 7000 Chur (CH); ACKERMANN, Armin, 8887 Mels (CH)
(74) Representative: EGLI-EUROPEAN PATENT ATTORNEYS

(56) References cited:
- EP-A1- 2 881 506
- EP-B1- 0 401 600
- EP-B1- 1 383 949

## Description

The present invention relates to a yarn separating module with a yarn separating device and a capacitive sensor device for monitoring the result of the yarn separation process. The invention further relates to a drawing-in machine as well as a warp tying machine or a leasing machine comprising at least one such yarn separating module.

The separation of yarns, in particular the separation of single yarns from a warp sheet, is an essential operational step necessary in many weaving preparation processes and systems, such as in warp tying or automatic drawing-in machines. Warp tying machines are used to knot a first yarn from an old warp sheet that has been woven in a weaving loom, with a second yarn from a new warp sheet that is to be woven in the weaving loom subsequently. The yarns of the two warp sheets are typically stretched in a so-called clamping frame or device, where the individual yarns lie very close to one another. Before tying, a single yarn first has to be separated from each respective warp sheet before the tying machine then knots the two separated yarns together and finally pulls the knotted yarns away. This process has to be repeated for all yarns of the two warp sheets. Drawing-in machines are used for the automatic drawing-in of warp yarns into corresponding elements (reed, dropwires, heddles) of a harness of a weaving machine. For this, the yarns are also arranged in at least one warp sheet stretched in a clamping frame. Before being drawn in, a predetermined number of yarns, in particular a single yarn, have to be separated from the warp sheet in preparation for the following operational steps.

Different solutions have been proposed for these separation processes. For example, EP 2 881 506 A1 describes a yarn separation device capable for separating yarns from a warp sheet by a rotating spindle device. Alternatively, EP 1 383 949 B1 discloses a separation device using a suction nozzle to catch a predetermined number of yarns from a warp sheet, in particular a single yarn.

In order to monitor the yarn separation result, i.e. in order to monitor the number of actually separated yarns with regard to the actually desired number of yarns to be separated by the yarn separation device, the separation device as disclosed in EP 2 881 506 A1 includes a detection device such as a camera or a tension measuring device. Though cameras may provide a lot of reliable or additional information, e.g. the number of yarns, color, type of yarn etc., they are quite expensive. Yet, tension measuring devices, such as piezo-electrical tension detectors, are often not fast enough for some applications.

Capacitive yarn detection is based on the modification of the electrical field of a capacitor in a capacitive sensor by a yarn placed within the measuring capacitor (i.e. between two electrodes), the modification depending on the yarn properties such as dimension, material etc. The capacitive sensor provides an output signal depending on the modified capacitance of the sensor. EP 0 401 600 B1 describes a capacitive sensor that is used in association with an optical sensor to detect irregularities along a yarn running in a gap-like monitoring volume between two plate electrodes of a capacitive sensor. When the yarn is running along its length extension through the gap-like monitoring volume of the capacitive sensor during detection, variations along the yarn length can be reliably detected. However, if yarns are successively brought into the gap-like monitoring volume, the results will be different depending on the actual position of the yarn within the monitoring volume. The structure of EP 0 401 600 B1 is thus not well adapted for capacitively monitoring the result of the separation process.

It is an object of the present invention to provide a yarn separating module with a fast, compact and non-expensive sensor device being capable to monitor the result of a separating process, in particular to provide reliable information about the number of yarns separated by the yarn separating device from a yarn layer or warp sheet, respectively.

This object is solved by a yarn separating module according to claim 1. Further aspects of the invention are subject to the dependent claims.

According to the invention, the yarn separating module comprises a yarn separating device and a capacitive sensor device for monitoring the yarn separation result, in particular the number of actually separated yarns. The yarn separating device is configured to separate a predetermined number of yarns, preferably a single yarn, from a yarn layer, and to transfer at least a length portion of the separated yarn(s) into a gap-like monitoring volume of a monitoring capacitor of the capacitive sensor device. The gap-like monitoring volume is formed between a first electrode and a second electrode of the monitoring capacitor which are facing each other and which are spaced transverse to a longitudinal direction of the length portion of the separated yarn(s) when extending through the monitoring volume of the monitoring capacitor. The monitoring capacitor and, thus, its monitoring volume, is configured such that it is open to the exterior of the capacitive sensor on both sides with regard to the longitudinal direction as well as in a direction opposite to an insert direction transverse to the longitudinal direction. This allows to insert a separated yarn or separated yarns to be monitored transverse to its/their length extension along the insert direction into the monitoring volume of the monitoring capacitor, there being arrangeable/placeable in a monitoring position, in which the yarn(s) pass(es) through the gap-like monitoring volume of the monitoring capacitor via both open sides with regard the longitudinal direction and is (are) supported by the two support surfaces such that at least the length portion of the yarn is arranged/placed within the monitoring volume of the monitoring capacitor. The insert direction is defined as a tangent of the path of the yarn inserted from the exterior of the capacitive sensor into the monitoring volume of the monitoring capacitor.

In order to place the yarn(s) in a fixed monitoring position within the monitoring volume of the monitoring capacitor, in particular relative to the first electrode and the second electrode, the capacitive sensor device further comprises at least two support surfaces for supporting the separated yarn(s). According to the invention, the two support surfaces are spaced along the longitudinal direction. According to the invention, the two support surfaces are fixed relative to the first electrode and the second electrode, i.e. they are stationary relative to the first electrode and the second electrode. The monitoring volume of the monitoring capacitor is arranged at least partially between the two support surfaces. In this way, when the separated yarn is supported by the support surfaces, the length portion of the separated yarn extending between the two support surfaces along the longitudinal direction intersects the monitoring volume of the monitoring capacitor. It influences thereby the electrical field of the monitoring capacitor. The two support surfaces are provided in addition to the electrodes. The two support surfaces are provided on the sensor device such that the support surfaces follow the advance movement of the separating device relative to the yarn layer. According to the present invention, it has been recognized that - in contrast to the capacitive detection of variations of a yarn parameter along its length extension, known from prior art, where the yarn is running through the gap-like volume of the capacitive sensor during detection - it is highly critical for reliably monitoring the result of a yarn separation process that each yarn to be monitored is held/arranged/placed in almost the same position within the monitoring volume of the monitoring capacitor of the capacitive sensor. This is due to the circumstances that for the monitoring of a yarn separation process, single yarns or groups of yarns are brought one after the other in the capacitive sensor device, causing the capacitive measurement - even if it is still the same kind of yarn - to be different for each yarn monitoring event, if there was no support delimiting the movement of the yarn(s) relative to the electrodes of the capacitive sensor at least in the insert direction. As described above with regard to the prior art, the yarns are typically over-tensioned during the separation processes. Hence, the sensor device is preferably arranged relative to the yarns to be monitored such that the over-tension is effective at least partially in the insert direction, causing the yarns to positively abut/rest against the two support surfaces under the over-tension load.

According to a preferred embodiment of the present invention, the at least two support surfaces may be configured such as to define a common plane that intersects the first and second electrode of the monitoring capacitor. The common plane is tangent to each support surface. Preferably each of the two support surfaces extends at least partially in the common plane. In that case, the intersection between each support surface and the common plane is at least a line transverse to the longitudinal direction in case the support surfaces are rounded, or at the most a portion of a plane in case the two support surfaces are flat and coplanar. Due to such a configuration of the support surfaces, the capacitive sensor device is compatible with different diameters of the yarn.

The monitoring volume and/or each of the first and second electrodes of the monitoring capacitor may extend on both sides of the common plane. This allows vibration movements of the yarn - when being supported on the support surfaces - at least in a direction perpendicular to the common plane - in particular in the monitoring volume, between the two support surfaces, within the electrical field of the monitoring capacitor. The vibration movements of the yarn induce a variation in the electrical field which can be measured and thus provide information about the yarn to be monitored, for example information about the tension of the yarn.

The common plane may intersect the first and the second electrodes of the monitoring capacitor at half height with regard to a respective height extension of the first and second electrodes, the height direction extending perpendicular to the longitudinal direction, especially perpendicular to the common plane.

Preferably, the first electrode and/or the second electrode of the monitoring capacitor extends at least over the full longitudinal spacing between the two support surfaces of the capacitive sensor device, such that a maximum of the length portion of the supported yarn extending between the two support surfaces is in the electrical field of the monitoring capacitor.

According to another embodiment of the present invention, the yarn separating device is configured to transfer a length portion of the separated yarn(s) into the monitoring volume of the monitoring capacitor in an insert direction. Each support surface may comprise a flat surface portion pointing opposite to the insert direction and being perpendicular to the insert direction.

According to yet another embodiment of the present invention, the first electrode and the second electrode of the monitoring capacitor may be configured such as to respectively define or may comprise a geometry respectively defining a first electrode plane and a second electrode plane, respectively. Each support surface may extend perpendicularly to the first electrode plane and/or to the second electrode plane.

The first electrode plane and the second electrode plane may either be parallel or inclined relative to each other. With inclined electrodes, the angle between the first electrode plane and the second electrode plane may have a value between 10° and 25°, preferably of 18°. In particular, the inclination is such that the two inclined electrodes converge in the insert direction. With inclined electrode planes, the monitoring capacitor is advantageously compatible with different yarn diameters. The capacitor is also well adapted for to- and fro-movements of the yarn perpendicular to the longitudinal direction. Inclination of the electrode planes may also allow compactness of the monitoring capacitor and of the capacitive sensor device: As the first and second electrode plates diverge in opposite direction of the insert direction, having a bell-mouthing effect, the monitoring capacitor and the capacitive sensor device in total can be arranged slightly tilted with regard to the insert direction. In order to further increase the compactness of the sensor device, the two support surfaces, and especially the common plane defined by the two support surfaces, may be substantially perpendicular to the first electrode plane or the second electrode plane. Furthermore, each support surface may comprise a flat surface portion parallel to the longitudinal direction of the yarn portion extending between the two support surfaces and transverse, in particular perpendicular, to the insert direction.

According to another embodiment of the present invention, each of the two support surfaces of the capacitive sensor device, and in particular the common plane defined by the two support surfaces of the capacitive sensor, is shifted from a plane in which a yarn layer is to be arranged relative to the separating device for yarn separation. In other words, each of the two support surfaces, and in particular the common plane, is positioned at a certain distance or spaced from the yarn layer plane in a direction perpendicular to the yarn layer plane.

According to another preferred embodiment of the present invention, the yarn separating module further comprises at least one abutment surface for the one or more yarns to abut against when extending through the monitoring volume of the monitoring capacitor. In combination with the two support surfaces, the at least one abutment surface may delimit a passage for at least a length portion of the yarn to be detected within the monitoring volume of the monitoring capacitor at a specific distance to the first and/or second electrode and to other parts of the monitoring capacitor and of the capacitive sensor device, respectively, thereby avoiding wear of those parts. Preferably, the abutment surface and the support surfaces are formed on metallic parts or are made of metal in order to make these surfaces well adapted for repetitive contact with the yarns. The abutment surface may define an abutment plane intersecting the two support surfaces or the common support plane, respectively, and extending at an angle between 90° and 105° with regard to the two support surfaces, preferably at an angle of 90°. The abutment plane extends between the first and second electrodes of the monitoring capacitor and is spaced from the first and second electrodes of the monitoring capacitor in a direction perpendicular to the abutment plane. Having an angle between the abutment surface and the support surfaces in this range, the yarns to be monitored may always come into contact with both, the support surfaces and the abutment surface, thus providing very reliable comparison results even for different yarns brought one after the other in the monitoring volume of the monitoring capacitor. When the yarn is in contact with the supports surfaces and the abutment surface, the yarn extends at distance from the first and second electrodes of the monitoring capacitor. Abutment for the yarns may be made by a flat front surface of the support also forming the at least one support surface. The edges of the abutment surfaces as well as the support surfaces may be rounded.

According to yet another aspect of the invention, the capacitive sensor device may further comprise a compensation capacitor, which preferably is substantially equal to the monitoring capacitor, in particular with regard to the electro-dynamical properties, such as capacitance, resistance, impedance, geometry etc. The compensation capacitor enables to consider and compensate environmental parameters like temperature, humidity etc., as it may serve as a reference to analyze the induced signal in the monitoring capacitor. The compensation capacitor also comprises a first electrode and a second electrode. The first electrode and the second electrode of the compensation capacitor form a gap-like monitoring volume therebetween. All electrodes of the capacitive sensor device are preferably aligned in a direction transverse to the longitudinal direction, in particular in a direction perpendicular to the longitudinal direction. In other words, the electrodes of the capacitive sensor are juxtaposed in a direction transverse to the longitudinal direction, in particular in a direction perpendicular to the longitudinal direction. The second electrode of the monitoring capacitor and the second electrode of the compensation electrode may be realized by a common electrode, which further increases the compactness of the sensor device and facilitates to make the compensation capacitor substantially equal to the monitoring capacitor.

According to another embodiment of the invention, the first electrode of the monitoring and/or the compensation capacitor comprises a preferably curved metallic conductive wire. Using a wire electrode increases sensitivity of the capacitor. Alternatively or additionally, the second electrode of the monitoring and/or the compensation capacitor may comprise a metallic conductive plate, respectively. Likewise, the common electrode may comprise a metallic conductive plate.

Furthermore, according to another embodiment of the invention, the first and/or the second electrode of the monitoring capacitor and/or the first and/or the second electrode of the compensation capacitor are held by an insulating member.

Preferably, all electrodes of the capacitive sensor are held by a common insulating member. The isolating member is typically made of plastics and provides insulation of each electrode from the others so that there is no contact between the electrodes.

According to another embodiment of the invention, at least one of the first electrode and the second electrode of the monitoring capacitor is formed by a metallic coating on an insulating member, preferably made of plastics. This metallic coating is electrically conductive. It fits the shape of the insulating member. Preferably the surface of the insulating member that is coated is a plane surface. Preferably, all electrodes (monitoring capacitor and compensation capacitor) of the capacitive sensor are formed by a metallic coating on a common insulating member. The isolating member provides insulation of each electrode from the others so that there is no contact between the electrodes.

According to another embodiment of the invention, the capacitive sensor device may comprise a casing. The casing surrounds or houses (at least partially) the monitoring capacitor with the first and second electrodes and - in case present - the insulating member and the compensation capacitor. The two support surfaces are formed by the casing in which an insulating member holding the first and second electrodes of the monitoring capacitor is fixed. The two support surfaces delimit the longitudinal sides of an insertion slot of the casing for inserting yarns from the exterior of the casing into the monitoring volume of the monitoring capacitor. In particular, when the monitoring volume of the compensation capacitor is placed within the casing, this allows for minimizing influence of other environmental parameters.

According to yet another embodiment of the invention, the two support surfaces are formed by the casing surrounding (at least partially) the monitoring capacitor with the first and second electrode and - in case present - the insulating member and the compensation capacitor. When the insulating member with the electrodes is fixed with the casing, the two support surfaces are fixed relative to the electrodes supported by the insulating member. Alternatively, the two support surfaces may be formed on the insulating plastic member. Forming the support surfaces on the casing provides the advantage that a material with higher hardness than material of the insulating member can be chosen so that wear caused by repetitive contacts with the separated yarns to be monitored is reduced.

Furthermore, the capacitive sensor device may comprise an electronic circuit operatively connected to the first and second electrodes of the monitoring capacitor and - in case present - to the first and second electrodes of the compensation capacitor. The electronic circuit is configured for generating a signal at one (sender) electrode of the capacitor and for the read-out and processing of signals the capacitive sensor provides based on the induced signal at the other (receiver) electrode of the corresponding capacitor. With regard to the monitoring capacitor, the induced signal depends on the number of yarn(s) within the monitoring volume of the monitoring capacitor. The electronic circuit may comprise a signal generator used to apply a voltage to the capacitor (s), in particular to the respective first (sender) electrode of the monitoring and the compensation capacitor, wherein each electrode can be supplied with a different voltage. The electronic circuit may further comprise a demodulator electrically connected to the generator and to the receiver electrode(s) of the monitoring and the compensation capacitors, a programmable gain amplifier (PGA) to provide an amplified analogic signal of the demodulated signal coming from the receiver electrode(s), and an analogic digital converter (ADC) to convert the amplified signal into an output digital signal. At least parts of the electronic circuit including the programmable gain amplifier (PGA) are housed in the casing of the capacitive sensor device. The signal generator, the demodulator, the PGA and/or the ADC are preferably also housed within the casing housing the monitoring capacitor, in order to shield sensitive components from perturbations and to reduce the need and thus the possible perturbative influence of long cabling. Advantageously, the PGA facilitates to increase the sensitivity of the capacitive sensor for compatibility with all types of yarns, in particular of different diameters.

In general, the capacitive sensor device according to the present invention may be configured for the detection of more parameters than absence/presence of a single yarn or a plurality of yarns. For example, it may be configured to provide further information about the yarn like yarn diameter (the larger the diameter, the higher induced signal), yarn tension (depending on yarn behavior within the monitoring volume of the monitoring capacitor, such as yarn vibrations which influence the induced signal).

Furthermore, according to an advantageous embodiment of the separating module, the separating device comprises at least one rotating spindle having an external helical groove, a back surface and a releasing edge at the intersection between the helical groove and the back surface. Preferably, the capacitive sensor device is placed at the back of the spindle with one of the first and the second electrode of the monitoring capacitor being placed inside the spindle. This helps to reduce size of the separating module and to directly transfer the separated yarns from the releasing edge into the monitoring volume of the monitoring capacitor.

Of course, the compensation capacitor may also be placed inside the spindle. Furthermore, a driving shaft of the spindle may extend through the capacitive sensor for compactness of the entire separating module.

Furthermore, a curved shape/portion of the (wired) first electrode of the monitoring capacitor may follow the curved circumferentially geometry of the spindle to detect the presence of yarn as soon as possible after the yarn is released from the spindle. This allows to stop the separating device for a second measurement (for determining the actual number of yarns released from the spindle and extending in the monitoring capacitor) before another yarn is released from the spindle into the monitoring volume of the monitoring capacitor. In addition, the curved shape/portion of the (wired) first electrode of the monitoring capacitor may extend in a plane perpendicular to an advance direction of the separating device relative to the yarn layer.

According to a yet another embodiment of the present invention, a back face of the rotation spindle may serve as abutment surface. Instead of by the back surface of the spindle, abutment for the yarn in the advance direction may be made by flat front surfaces of supports that form the two support surfaces for the yarn extending in the monitoring capacitor, i.e. by two abutment surfaces for the yarn in the advance direction. These abutment surfaces on the supports may be rounded. The angle between a plane formed by the abutment surface (s) and the support surfaces may be between 90° and 105°.

Alternatively, the separating device may comprise a suction nozzle, e.g. as described in EP 1 383 949 B1. In that case, the separated yarn is separated from the sheet by the nozzle and brought into the monitoring volume of the monitoring capacitor in contact with the two support surfaces. If the suction nozzle deflects the yarn within the warp sheet plane, the insert direction of the separated yarn into the monitoring volume of the monitoring capacitor also extends in the warp sheet plane. Contact with the support surfaces may be maintained by the nozzle which still sucks the yarn.

The present invention further relates to a warp drawing-in machine comprising at least a yarn separating module according to the present invention, as described above. Preferably, a controller of the drawing-in machine is operatively connected to the capacitive sensor device of the at least one yarn separating module. The controller may also control a drive of a gripper that inserts the separated yarn into harness elements of a weaving machine.

In addition, the present invention relates to a warp tying machine for joining yarns from two different yarn layers, the warp tying machine comprising a respective yarn separating module according to the invention for each of the two yarn layers. Preferably, a controller of the warp tying machine is operatively connected to the capacitive sensor device of each yarn separating module. The controller of the warp tying machine may be also operatively connected to the separating device.

The warp tying machine according the invention may further comprise a yarn transfer device associated to each yarn separating module for taking out yarn(s) from the monitoring volume of the sensor device, the controller of the warp tying machine is operatively connected to a drive of the yarn transfer device.

The capacitive sensor device may also be used for monitoring separation results of a leasing machine and in general of all machines that have to separate yarns from a yarn layer. In that case the separation result coming from the capacitive sensor is an input of the separating device control.

The invention will now be further described by way of an example only with reference to the accompanying drawings in which:
- Fig. 1: shows an example of warp tying machine with two separating modules each equipped with a capacitive sensor device according to a first or a second exemplary embodiment of the present invention;
- Fig. 2: shows an exploded view of the separating module according to a first exemplary embodiment;
- Fig. 3: shows an exploded view of the electrodes arrangement of the capacitive sensor according to Fig. 2;
- Fig. 4: shows a section view of the separating module according to Fig. 2;
- Fig. 5: shows a cross view of the arrangement according to Fig. 2 along the cross line C in Fig. 4;
- Fig. 6: shows a section view of a separating module according to a second exemplary embodiment;
- Fig. 7: shows an isometric view of the electrode arrangement of the second exemplary embodiment according to Fig. 6;
- Fig. 8: shows a cross view of the separating module according to Fig. 6 along the cross line C in Fig. 6;
- Fig. 9: shows a schematic electrical diagram of the tying machine according to Fig. 1 with two capacitive sensors and a tying machine main controller; and
- Fig. 10: shows an isometric view of the yarn separating module according to a third exemplary embodiment.

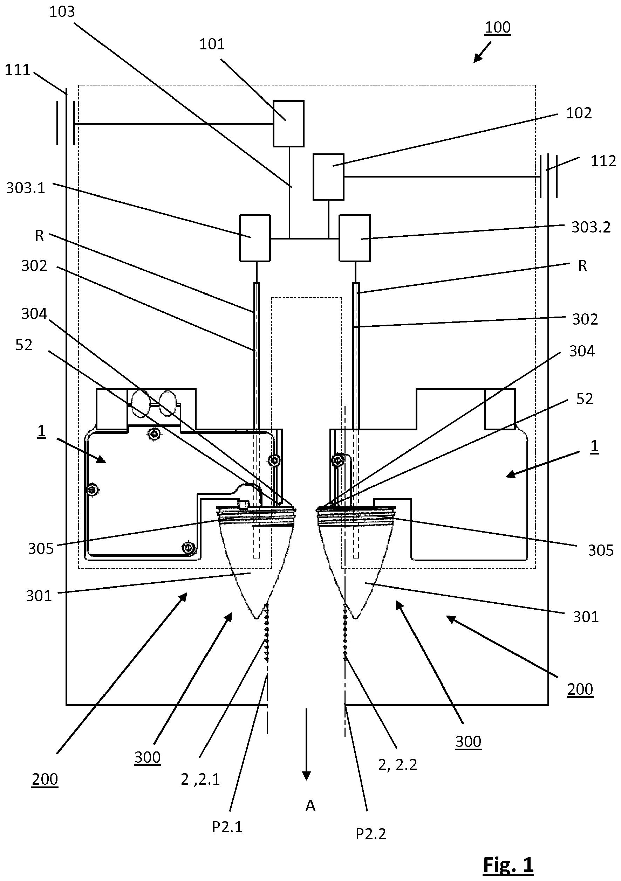

Fig. 1 shows an example of warp tying machine 100 with two separating modules 200 each equipped with a separating device 300 and a capacitive sensor device 1 according to an exemplary embodiment of the present invention. Before tying process, yarns 2 of an upper warp sheet 2.1 and a lower warp sheet 2.2 are clamped and tensioned in a upper and lower clamping device 111, 112 so that upper warp sheet 2.1 and a lower warp sheet 2.2 are separated from each other. Upper warp sheet 2.1 and lower warp sheet 2.2 respectively defines a plane P2.1 and a plane P2.2, respectively. The purpose of the warp tying machine 100 is to separate a yarn 2 from the upper warp sheet 2.1 and a yarn 2 from the lower warp sheet 2.2 and to make a join (knot) between the two separated yarns 2. This shall be repeated for all yarns 2 of the sheets 2.1, 2.2. The warp tying machine 100 is put on the clamping devices 111, 112 with two of its drives 101, 102 controlling relative position between a tying machine frame 103 and the warp sheets 2.1, 2.2 (in an advance direction A of the warp tying machine 100 relative to the warp sheets 2.1, 2.2).

The warp tying machine 100 comprises two separating modules 200, each including a respective separating device 300. Each separating device 300 includes a single spindle 301 with an external helical groove 305 driven by a dedicated drive 303.1, 303.2 via a rotating spindle shaft 302 around a rotating axis R. The spindle 301 has a back surface 306 at the opposite of the warp sheet 2.1, 2.2 along the advance direction A and a releasing edge 304 at the intersection of the root of the helical groove 305 and the back surface 306. Such a separating device is basically known from prior art, e.g. from EP 2 881 506 A1. Furthermore, each separating module 200 includes a capacitive sensor device 1 according to the present invention which is used in association with a respective separating device 300 for capacitively monitoring the separation results, i.e. to determine whether the separation process of the respective separating device 300 provides a predetermined number of yarns to be separated. Hence, in case a single yarn is to be separated, the capacitive sensor device 1 allows for checking whether a separation process yielded one or more than one yarn.

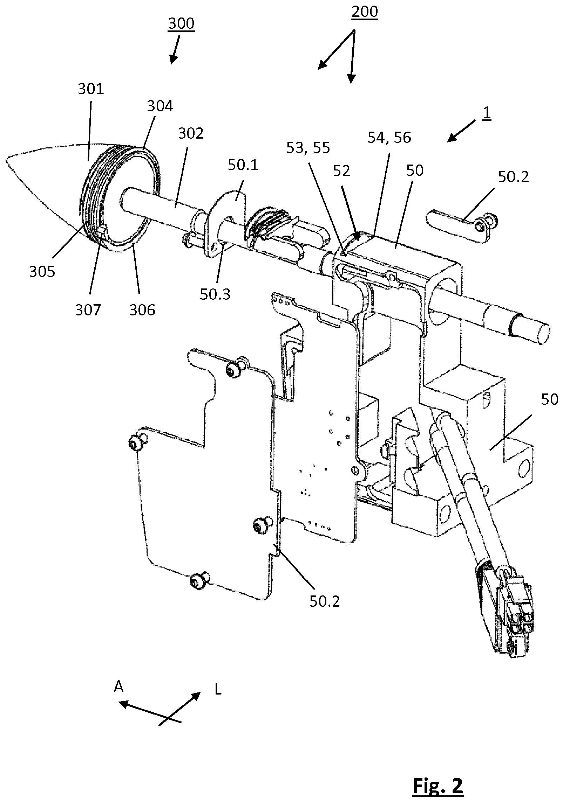

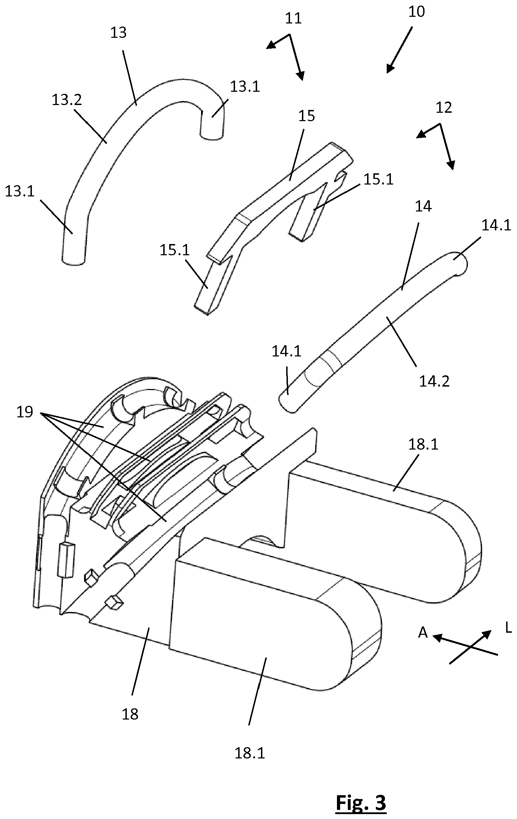

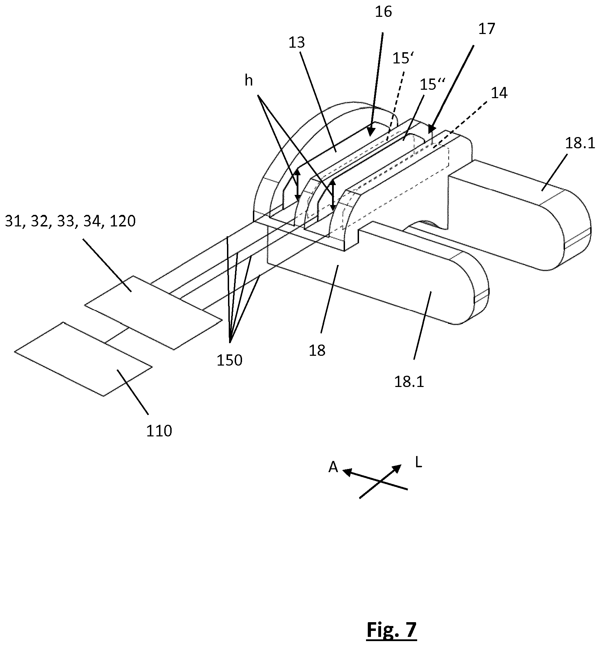

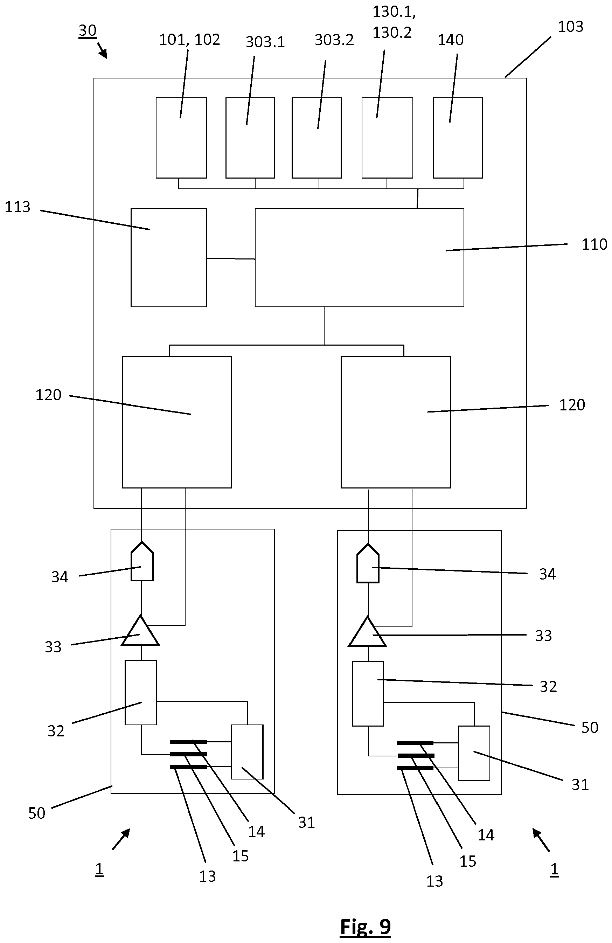

Each capacitive sensor device 1 basically comprises an electrode arrangement 10 (in particular see Fig. 3 or Fig. 7), an electronic circuit 30 (in particular see Fig. 9), and a casing 50 (in particular see Fig. 2).

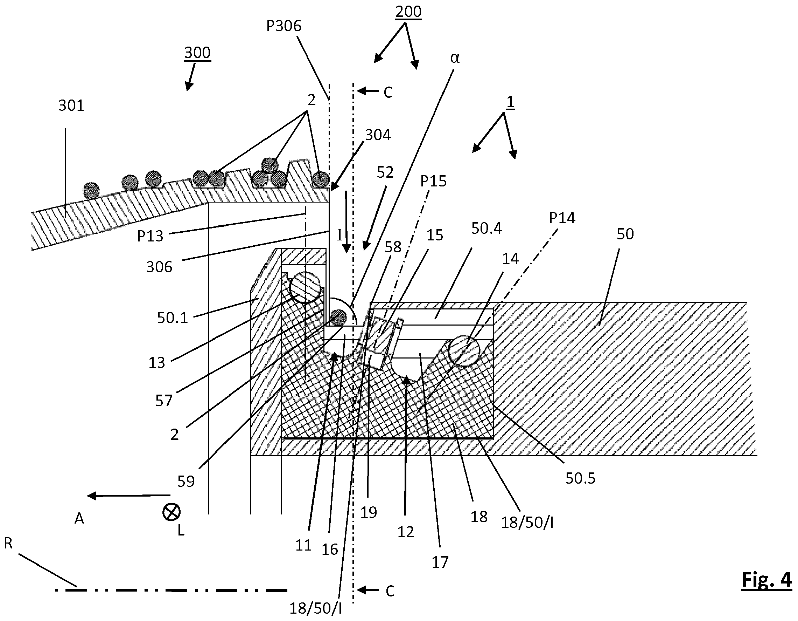

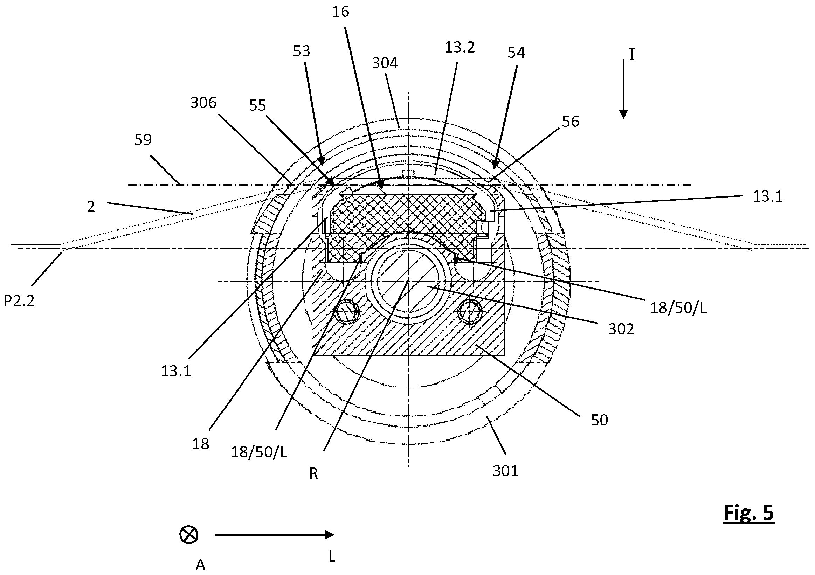

A first embodiment of the capacitive sensor device 1 is now explained in more detail with reference to Figs. 2-5. In particular Fig. 3 illustrates the electrode arrangement 10 in detail. The capacitive sensor device 1 comprises two capacitors: a monitoring capacitor 11 and a reference or compensation capacitor 12. In the present embodiment, both capacitors 11, 12 comprise a first (sender) electrode 13, 14 formed by a curved metal wire. The sender electrode wires have a circular cross section (same cross section for both first electrodes 13, 14). Furthermore, both capacitors 11, 12 have a common metal plate electrode 15 (receiver electrode) representing the respective second electrode of both capacitors 11, 12. The plate electrode 15 defines two opposite flat surfaces which faces the first electrode 13 and the first electrode 14, respectively, along a direction transverse to the longitudinal direction L and along a direction transverse to the insert direction I. The first wire electrode 13 (sender electrode) of the monitoring capacitor 11 and the (common) plate electrode 15 are spaced from one another to form therebetween an elongated gap-like monitoring volume 16 of the monitoring capacitor 11 in which a length portion of a yarn can extend along a longitudinal direction L. The first wire electrode 14 (sender electrode) of the compensation capacitor 12 and the (common) plate electrode 15 are also spaced from one another transverse to a direction parallel to the longitudinal direction L to form a gap-like monitoring volume 17 therebetween, which belongs to the compensation capacitor 12 and which is elongated in a direction parallel to the longitudinal direction L.

All electrodes 13, 14, 15 are held by a common insulating member 18 such that electrodes 13, 14, 15 are stationary relative to the insulating member 18. The electrodes 13, 14, 15 are aligned in a direction transverse to the longitudinal direction L. The insulating member 18 is made of plastics and spaces each electrode 13, 14, 15 from the others so that there is no contact between the electrodes 13, 14, 15. The gap-like monitoring volume 16 of the monitoring capacitor 11 only contains air in case no yarn extends therethrough. The electrical field between the first and second electrode 13, 15 permeates the gap-like monitoring volume 16. The electrical field between the first and second electrode 14, 15 permeates the gap-like monitoring volume 17. The insulating member 18 comprises corresponding supporting grooves 19 for each of the electrodes 13, 14, 15 to be held by. The two wire electrodes 13, 14 have basically the same curved shape and comprise two lateral arms 13.1, 14.1 to engage with the insulating member 18, and a central curved portion 13.2, 14.2. The plate electrode 15 also includes two lateral arms 15.1 to be held in the insulating member 18.. Furthermore, the insulating member 18 comprises two back flanges 18.1 for positioning and mounting the insulating member 18 within a housing 50.4 of the casing 50.

The curved shape/geometry of the metal wire of the first (sender) electrode 13 of the monitoring capacitor 11 defines a median plane P13 which is inclined relative to a median plane P15 defined by the plate of the common electrode 15. The same angle occurs between a median plane P14 defined by the curved wire electrode 14 of the compensation capacitor 12 and the plane P15 of the plate electrode 15. Plane P13 of the electrode 13, plane P15 of the electrode 15 and plane P14 of the electrode 14 are parallel to each other. In the present example, the angle between each wire plane P13, P14 and plate plane P15 is around 18°, preferably between 10° and 25°. The plane P13 of the electrode 13 is perpendicular to the advance direction A and to the rotating axes R.

The monitoring volume 16 of the monitoring capacitor 11 - when electrodes 13, 15 are held by the insulating member 18 in the casing 50 - communicates with the exterior of the capacitive sensor 1 on both sides with regard to the longitudinal direction L. The exterior of the electrode arrangement 10 also communicates with monitoring volume 16 in an insert direction I transverse to the longitudinal direction L and extending between the two electrodes 13, 15 of the monitoring capacitor 11. The insulating member 18 closes the monitoring volume 16 on the other side along the insert direction I. The insert direction I is directed from the exterior of the capacitive sensor 1 into the monitoring volume 16. In the described embodiment with the separating device 300 comprising the spindle 301, the insert direction I is vertical and perpendicular to the associated warp sheet plane P2.1, P2.2. In particular, the insert direction I is perpendicular to the advance direction A. This allows the separating device 300 to transfer a yarn or several yarns 2 to be monitored transverse to its/their length extension in the insert direction I into the monitoring volume 16, there being arrangeable/placeable in a monitoring position, in which the yarn(s) 2 to be monitored pass (es) through the gap-like monitoring volume 16 via both open sides along the longitudinal direction L such that at least a length portion of the yarn 2 is arranged/placed within the monitoring volume 16. The insulating member 18 closes the monitoring volume 16 of the monitoring capacitor 11 and the monitoring volume 17 of the compensation capacitor 12 in the direction of convergence of the electrode planes.

The casing 50 of the capacitive sensor device 1 houses the electrodes 13, 14, 15, the insulating member 18, and at least parts of an electronic circuit 30 for the read-out of the capacitive sensor device 1. The casing is preferably made of metal, for example aluminum or steel. Furthermore, the casing 50 has a through-hole 51 which the spindle shaft 302 driving the spindle 301 extends through. The casing 50 further comprises an insertion slot 52 through which the exterior of the casing 50 can communicate with the interior of the casing 50.

Two supports 53, 54 of the casing 50 form the longitudinal sides of the slot 52. These two supports 53, 54 also form two support surfaces 55, 56, that are provided according to the invention for supporting one or more yarns 2 to be monitored within the monitoring volume 16 of the monitoring capacitor 11. Accordingly, both supports 53, 54 are spaced in the longitudinal direction L, each having at least one support surface 55, 56 fixed relative to the first (sender) and second (common) electrode 13, 15 of the monitoring capacitor 11, when the capacitive sensor device 1 is readily assembled, with the monitoring volume 16 extending at least partially between the two support surfaces 55, 56 along the longitudinal direction L. Each support surface 55, 56 forms the bottom of a U-shaped opening formed by the supports 53, 54 and points in a direction opposite to the insert direction I for supporting yarns 2 at least in the insert direction I. The U-shaped openings of the supports 53, 24 are open to the exterior of the casing 50 in a direction opposite the insert direction I.

In addition to the respective flat support surface 55, 56, each support 53, 54 has a flat front surface 57 and a flat back surface 58, which all together delimit the U-shaped cross section opening of the supports 53, 54 in a plane transverse, and in particular perpendicular, to longitudinal direction L. In the longitudinal direction L, both support surfaces 55, 56 are arranged to one another as close as possible. Preferably, the longitudinal distance between the two supports 53, 54 is less than the external diameter of the spindle 301 at the releasing edge 304. In the present example, the longitudinal distance between the two supports 53, 54 amounts about 12 mm. The width (along the advance direction A) of each support surface 55, 56 is preferably larger than the biggest diameter of a yarn to be detected by the capacitive sensor device 1. Typical yarn diameters are on the order of 1 mm. Therefore, the width of each support surface 55, 56 amounts about 1.5 mm in the present example. Each flat front surface 57 intersects the respective support surface 55, 56 of each support 53, 54. The angle α between the flat front surface 57 and the flat support surface 55, 56, measured in the U-shape opening of the supports 53, 54, is 90° in the present example, but may be in general any angle between 90° and 105°. The casing 50, the insulating member 18 and the electrodes 13, 14, 15 form a measuring head of the capacitive sensor device 1.

Fig. 9 exemplary illustrates the electronic circuit 30 of the capacitive sensor device 1 as it is in general known from EP 0 129 076 B1 which also discloses an example of electronic processing of the induced signal in a capacitive sensor device 1. The electronic circuit 30 is operatively connected to the electrodes 13, 14, 15. The capacitive sensor device 1 is wired to an energy supply (not shown). The electronic circuit 30 comprises a signal generator 31 used to apply a voltage to each first (sender) electrode 13, 14 (each electrode can be supplied with a different voltage) of the monitoring and the compensation capacitor 11, 12, respectively, a demodulator 32 operatively connected to the generator 31 and to the common receiver electrode 15, a programmable gain amplifier 33 (PGA) to provide an amplified analogic signal of the demodulated signal coming from the receiver electrode 15, an analogic digital converter 34 (ADC) to convert the amplified signal coming from the programmable gain amplifier 33 into an output digital signal. The signal generator 31, the demodulator 32, the PGA 33 and the ADC 34 are housed within the casing 50 of the capacitive sensor device 1.

Due to the electronic circuit 30 connected to the electrodes 13, 14 15, the capacitive sensor device 1 is capable to provide a signal depending on the induced signal at the receiver electrode 15 and thus depending on the yarn(s) in the monitoring volume 16 during measurement of the induced signal which allows to capacitively monitor the presence and the number of the yarns in the monitoring volume 16. The compensation capacitor 12 is used for reference of the induced signal, independently of environmental parameters, e.g. ambient temperature, humidity, etc.

Turning back to the assembly of the first embodiment of the capacitive sensor device 1 as shown in Fig. 2, 3, 4 and 5, the insulating member 18 is held in position within the casing 50 by the back flanges 18.1, preferably via at least two abutments 18/50/L in opposite directions along the longitudinal direction L and two abutments 18/50/1 in opposite directions along a direction perpendicular to longitudinal direction L and to advance direction A. A front cap 50.1 closes (e.g. with screws) the front opening of the casing 50 through which the insulating member 18 equipped with the electrodes 13, 14, 15 is introduced into the casing 50 and holds the insulating member 18 sandwiched between a shoulder 50.5 of the casing (backwards) and the front cap 5.1 along the advance direction A. Two opposite lateral caps 50.2 close the opposite lateral openings of the casing 50 (e.g. by screws) and achieve the correct positioning of the insulating member 18 relative to the casing 50 in the longitudinal direction L. With this assembly, the insulating member 18 and, thus the electrodes 13, 14, 15 are accurately fixed relative to the casing 50 and, thus, to the two supports 53, 54. The front cap 50.1 has a through-hole 50.3 to enable the driven shaft 302 of the spindle 301 to pass through. Except the through-hole 51 and the slot 52, the casing 50 is closed.

The two support surfaces 55, 56 of the supports 53, 54 define together a common plane 59 that is tangent to both support surfaces 55, 56 (and parallel to longitudinal direction L) and that partially extends in the monitoring volume 16 of the monitoring capacitor 11. The two flat support surfaces 55, 56 extend in the common plane 59. The common plane 59 extends between the two supports 53, 54 and between the two support surfaces 55, 56 and is tangent to the length portion of the yarn 2 that bears on the two support surfaces 55, 56. The common plane 59 is perpendicular to the plane P13 of the first (sender) electrode 13 of the monitoring capacitor 11. Generally, each support surface 55, 56 extends with an angle of 90° to 115° with the plane P13 of the first electrode 13 or with the plane P15 of the second electrode 15. Advantageously, each support surface 55, 56 extends in a direction perpendicular to the insert direction I. The common plane 59 defined by the support surfaces 55, 56 is also inclined relative to the plane P15 of the plate-like common electrode 15. The plane defined by each of the flat support surfaces 55, 56 intersects both, the first electrode 13 and the common electrode 15 of the monitoring capacitor. In particular, the common plane 59 intersects both the first electrode 13 and the second electrode 15 of the monitoring capacitor 11. Monitoring volume 16 is partially open to the exterior of the capacitive sensor 1 in the two longitudinal sides via the U-openings of the supports 53, 54 of the casing 50.

The casing 50 forms a shielding around the insulating member 18, the external part of the curved wire of the first electrode 13 of the monitoring capacitor 11, the external part of the plate-like common electrode 15, the monitoring volume 17 of the compensation capacitor 12, the external part of the curved wire 14 of the compensation capacitor 12 and parts of the electronic circuit 30. The gap-like monitoring volume 17 of the compensation capacitor 12 only contains air, whereas the gap-like monitoring volume 16 of the monitoring capacitor 11 contains air and is configured to receive yarn(s) to be monitored, desirably one after the other. The monitoring volume 16 of the monitoring capacitor 11 is partially open to the exterior of the casing 50 along the insert direction I transverse to the longitudinal direction L via the slot 52 of the casing 50. The monitoring volume 17 of the compensation capacitor 12 is closed to the exterior of the casing 50 along the insert direction I by the casing 50.

The two supports 53, 54 are placed very close to the electrodes 13, 14, 15 along the longitudinal direction L. In other words, the first electrode 13 extends from the longitudinal level of one of the support surface 55 to the longitudinal level of one of the other support surface 56 and the second electrode 15 extends from the longitudinal level of one of the support surface 55 to the longitudinal level of one of the other support surface 56 so that the maximum of the length portion of supported yarn(s) extending between the two supports 53, 54 is within the electrical field of the monitoring capacitor 11. In other words, the first electrode 13 of the monitoring capacitor 11 extends at the level of each of the two support surfaces 55, 56 and continuously between the two support surfaces 55, 56 and the second electrode 15 of the monitoring capacitor 11 extends at the level of each of the two support surfaces 55, 56 and continuously between the two support surfaces 55, 56. Furthermore, the monitoring volume 16, the first electrode 13, and the second electrode 15 extend on both sides of the common plane 59 defined by the two support surfaces 55, 56 in a direction perpendicular to the common plane 59.

According to the invention, the capacitive sensor device 1 may be directly associated to a spindle separating device 300, together forming a separating module 200 that can be introduced into a warp tying machine 100 in order to separate a predetermined number of yarns, in particular single yarns, and to monitor the number of actually separated yarns being brought into the sensor device 1 and removed therefrom one after the other. Referring to the separating module 200 according to the first embodiment shown in Fig. 1, 2, 4 and 5, the separating module 200 moves relative to the warp sheet in the advance direction A directed towards the warp sheet. The capacitive sensor device 1 is placed at the back surface 306 of each spindle 301 so that a yarn transported in the helical groove 305 and falling from the releasing edge 304 of the spindle 301 may be guided by the back surface 306 to directly enter (at least partially/portion-wise) into the monitoring capacitor 11 of the capacitive sensor device 1 via the insertion slot 52 of the casing 50. Insert direction I of the length portion of the separated yarn is thus parallel to spindle back surface plane P306. As yarns 2 are deflected by the spindle 301 for the separation process and as each yarn is still deflected in a direction perpendicular to the respective warp sheet plane P2.1, P2.2 when resting on the support surfaces 55, 56, the transfer of yarns by the spindle 301 occurs directly into the monitoring volume 16 without additional transport means, only due to deflection of the yarn 2. The longitudinal direction L of the length portion of the yarn 2 - when extending through the monitoring volume 16 of the monitoring capacitor 11 - is parallel to length extension of the yarns 2 before being separated by the spindle 301, i.e. in the warp sheets 2.1, 2.2. The longitudinal direction L of the length portion of the yarn 2 - when extending through the monitoring volume 16 - is also orthogonal to the advance direction A and to the rotating axis R of the spindle 301. In particular, in each separating module 200, the first (sender) electrode 13 of the monitoring capacitor 11 is placed inside the spindle 301, i.e. is entirely placed in the interior of the spindle 301 regarding to the back surface plane P306, with the external helical groove 305 at the level of the first electrode 13 along the rotating axis R, with its curved portion 13.2 as close as possible to the releasing edge 304. Only one of the electrode 13 of the monitoring capacitor 11 is placed inside the spindle 301, whereas the other electrode 15 of the monitoring capacitor 11 is placed outside the spindle 301. In particular, the axis of the curved portion 13.2 of the first electrode 13 of the monitoring capacitor 11 coincides with the rotating axis R of the rotating shaft 302 of the spindle 301. The gap-like monitoring volume 16 between the common electrode 15 and the first electrode 13 of the monitoring capacitor 11 is open to the exterior in a direction opposite to the insert direction I, the latter being orthogonal to the longitudinal axis L and orthogonal to the rotating axis R of the spindle 301. The gap-like monitoring volume 16 is also open with regard the exterior of the electrode arrangement 10 in the backward direction, i.e. opposite of the warp sheet 2.1, 2.2 and opposite of the back surface 306 of the spindle 301, due to the angle of 18° between the planes of the first electrode 13 and the common electrode 15 of the monitoring capacitor 11. The front surface 57 of each support 53, 54 is placed inside the spindle 301 so that, along the advance direction A, a plane P306 passing through the back face 306 of the spindle 301 intersects the two support surfaces 55, 56 of the supports 53, 54, in the present case at an angle α equal to 90°, preferably at angle α between 90° and 105°, as well as the monitoring volume 16. In particular, the plane P306 extends between the electrodes 13, 15 at distance from the electrodes 13, 15 of the monitoring capacitor 11 in a direction perpendicular to abutment plane P306 and is placed, along the advance direction A, between the warp sheet 2.2, 2.3 and the yarn 2 extending in the monitoring volume 16. The plane P306 is parallel to plane P13. In particular, the plane P306 is shifted along the advance direction A from the yarn 2 lying at the edge of the warp sheet 2.2, 2.3 so that a yarn 2 supported by the two support surfaces 55, 56 comes in abutment with the back face 306 which serves as abutment surface.

According to the present exemplary embodiment shown in Fig. 1, the warp tying machine 100 comprises two separating modules 200, one separating module 200 for an upper warp sheet 2.1, the other separating module 200 for a lower warp sheet 2.2, wherein the respective slots 52 in the casings 50 of those separating modules 200 are face to face. Furthermore, the respective casings 50 are fixed to the frame 103 supporting the respective separating device 300, so that they follow the advance movement of the associated separating device 300 relative to the warp sheet 2.1, 2.2.

The plane P13 defined by the first (sender) wire electrode 13 of the monitoring capacitor 11 is vertical (perpendicular to the horizontal plane P2.1, P2.2 of the warp sheets 2.1, 2.2) when the tying machine 100 is in working position on the clamping devices 111, 112.

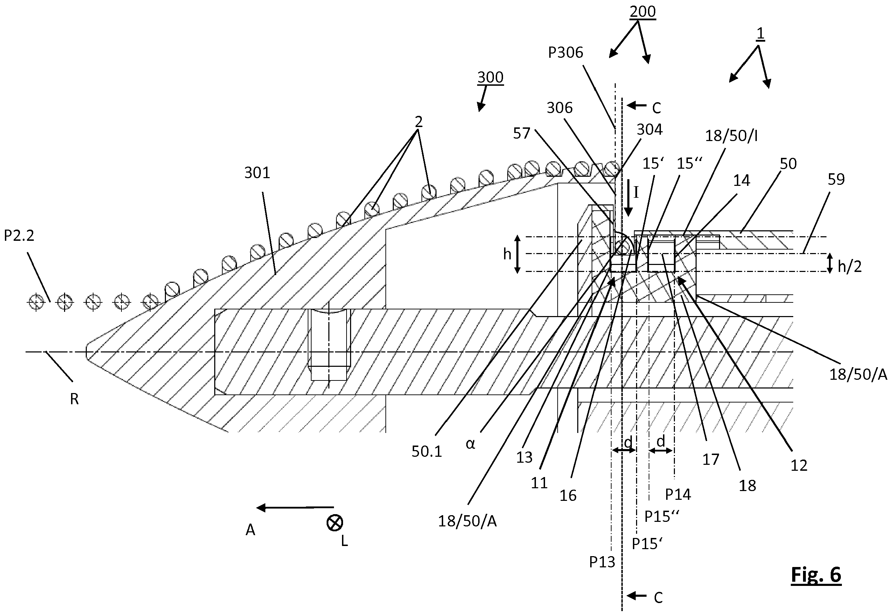

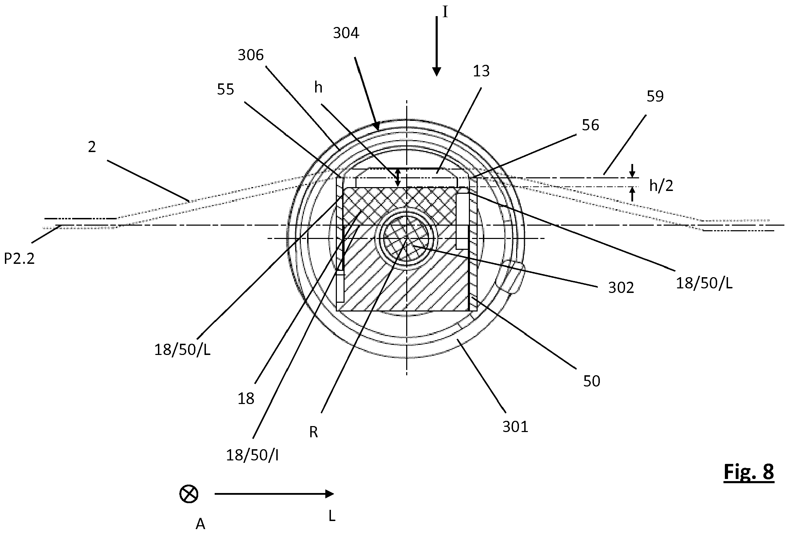

A second embodiment of the capacitive sensor device 1 is now explained in more detail with reference to Figs. 6-8. The basic concept of the capacitive sensor device 1 according to the second embodiment is very similar to the capacitive sensor device according to the first embodiment shown in Figs. 2-5. Therefore, identical features are denoted with identical reference numerals unless otherwise explicitly indicated and are not explained in detail.

Analogous to the first embodiment shown in Figs. 2-5, the capacitive sensor device 1 according to the second embodiment is placed at the back surface 306 of a spindle 301 with helical groove 305 and releasing edge 304. During insertion into the monitoring volume 16 along the insert direction I upon the release of the yarns 2 from the spindle 301, each yarn 2 comes into contact with the back face 306 of the spindle 301, which constitutes an abutment surface 306, in the advance direction A.

In contrast to the first embodiment according to Fig. 2-5, the capacitive sensor device 1 according to the second embodiment comprises a monitoring capacitor 11 and a compensation capacitor 12 having no common second electrode but each having a separate second electrode 15', 15".

Further in contrast to the first embodiment according to Fig. 2-5, the capacitive sensor device 1 according to the second embodiment does not comprise wire-like first electrodes. Instead, the capacitive sensor 1 comprises an insulating member 18 made of plastic which includes two parallel grooves parallel to the longitudinal direction L. Each of the grooves defines two parallel surfaces facing each other along a direction transverse to the longitudinal direction L and along a direction transverse to the insert direction I, which are partially coated with a metallic coating which is electrically conductive to form the respective first and second electrodes 13, 15'; 14, 15" of the monitoring capacitor 11 and the compensation capacitor 12. The metallic coatings of the first groove facing each other define the monitoring volume 16 of the monitoring capacitor 11, while the metallic coatings of the second groove facing each other define the monitoring volume 17 of the compensation capacitor 12.

The first electrode 13 and second electrode 15' of the monitoring capacitor 11 as well as the first electrode 14 and second electrode 15" of the compensation capacitor 12 define median planes P13, P15', P14, P15" parallel to each other and are aligned in a direction transverse to the longitudinal direction L, for example in a direction parallel to the advance direction A. The first electrode 13 is placed inside the spindle 301, the electrodes 15', 15" and 14 outside the spindle 301.

The thickness of the metallic coating and thus the thickness of the first and second electrodes 13, 15'; 14, 15" of the monitoring capacitor 11 and the compensation capacitor 12 may preferably be between 0.2mm and 0.6mm. The shape of the metallic coating and thus the shape of the first and second electrodes 13, 15'; 14, 15" of the monitoring capacitor 11 and compensation capacitor 12 as seen in the advance direction A may be quadratic, rectangular, trapeze-like or a combination thereof, for example including a rectangular portion and a contiguous trapeze-like portion. The coating may be directly applied to the parallel surfaces of the grooves. The surfaces of the grooves facing each other may each include a recess for the coating to be applied thereon. In that case, the depth of the recess preferably corresponds to the thickness of the coating applied thereon. In any case, having a metallic coating directly applied to the insulating member 18 avoids any disadvantages due to air gaps. The geometry of the metallic coatings is preferably the same for all electrodes 13, 14, 15', 15" of the monitoring capacitor 11 of the compensation capacitor 12.

Moreover, a distance d between the first and second electrodes 13, 15' of the monitoring capacitor 11 along the advance direction A is equal to a distance d between the first and second electrodes 14, 15" of the compensation capacitor 12 along the advance direction A.

The metallic coating of each electrode 13, 15', extends on both sides of the common plane 59 defined by the flat support surfaces 55, 56 formed on the metallic casing 50 with regard to a direction perpendicular to the common plane 59. The flat support surfaces 55, 56 extend perpendicularly to planes P13, P15' and point opposite to the insert direction I. Each coating extends from the bottom of the respective groove up to a height extension h, the height direction being perpendicular to the common plane 59 and parallel to the insert direction I. In particular, the common plane 59 may intersect or bisect the metallic coating of the first and second electrodes 13, 15', of the monitoring capacitor 11 at half height h/2 with regard to the height extension h of each electrode 13, 15'. The common plane 59 is spaced from or extends at certain distance from the bottom of the groove defining the monitoring capacitor. At the distance from the edges of the electrodes 13, 14, 15', 15" in the height direction, the electrical field is more regular. As can be seen from Fig. 6, the width of the support surfaces 55, 56 in the advance direction A is smaller than the distance between the first and second electrodes 13, 15' of the monitoring capacitor 11.

For mounting in the casing 50, the measuring head, including the insulating member 18 equipped with the electrodes 13, 14, 15', 15", may engage with the casing 50 via abutments, preferably via at least two abutments 18/50/L in opposite directions along the longitudinal direction L, two abutments 18/50/A in opposite directions along the advance direction A and two abutments 18/50/1 in opposite directions along a direction perpendicular to the longitudinal direction L and to the advance direction A (for example along the insert direction I) .

As can be seen from Fig. 6, the common plane 59 is shifted relative to the warp sheet plane P2.2, in which the yarn layer 2.2 is arranged relative to the yarn separating device 300 with spindle 301. In particular, the common plane 59 is placed between the plane P2.2 and a parallel plane passing through the releasing edge 304, the rotation axis R of the spindle 301 being out of the volume delimited between the warp sheet plane P2.2 and the parallel plane passing through the releasing edge 304.

As can be seen from Fig. 7, electrical connections 150 between the electrodes 13, 14, 15', 15" and the electronics 31, 32, 33, 34, 120, 110 are preferably arranged at one longitudinal side of the insulating member 18 and fixed to electrodes 13, 14, 15', 15" for example by soldering.

With regard to the second embodiment of the capacitive sensor device 1 shown in Figs. 6-8, it is to be noted that the demodulator 32 is operatively connected to both, the second electrode 15' of the monitoring capacitor 11 as well as to the second electrode 15" of the compensation capacitor 12 (not shown in Fig. 9).

Referring again to Fig. 9, a main controller 110 of the tying machine 100 controls the upper sheet drive 101 and the lower sheet drive 102, the spindle drive 303.1 for rotating the separating device 300 for the upper warp sheet 2.1, the spindle drive 303.2 for rotating the separating device 300 for the lower warp sheet 2.2, a transfer and cutter drive 130.1 for a yarn of the upper warp sheet 2.1, a transfer and cutter drive 130.2 for a yarn of the upper warp sheet 2.2 and a knotting unit drive 140. The main controller 110 is operatively (directly or indirectly) connected to each capacitive sensor device 1. The digital signals provided by each ADC 34 of the upper and lower warp sheet 2.1, 2.2 are analyzed by a dedicated signal processor 120 of the capacitive sensor 1 which is housed in the tying machine frame 103. A pluggable/unpluggable electric connection between the electronics housed in casing 50 and electronics housed in frame 103 is realized by the connector element (shown on Figure 2) and a complementary connector element (not shown). Alternatively, the signal processor 120 can be housed in casing 50 of the capacitive sensor device 1. Each signal processor 120 provides information about the presence or absence of yarn(s) 2 and/or information about the number of yarn(s) 2 in the monitoring volume 16 of the monitoring capacitor 11 of the respective separating module 200. Each signal processor 120 may dynamically act on the associated PGA 33 for modification of the amplification provided by the PGA 33. Hence, in order to optimize the analysis, the signal processor 120 may increase the amplification at the PGA 33 as soon as the signal transmitted by the PGA 33 is of small amplitude, in order to use the ADC 34 in its optimal range. Analog/digital conversion is used when needed. Each signal processor 120 is electrically and operatively connected to the main controller 110 of the tying machine 100.

During separation of the yarns 2 from the warp yarn sheets 2.1, 2.2, exciting signals are regularly sent to sender electrodes 13, 14 of capacitors 11, 12 by the signal generators 31, wherein the exciting signals for the monitoring capacitor 11 is phase-shifted, i.e. opposite with regard to the exciting signal for the associated compensation capacitor 12. When a yarn 2 enters the monitoring volume 16 of a monitoring capacitor 11, the induced signal at the common receiver electrode 15 or at each of the second electrodes 15', 15" is modified and further processed through the demodulator 32, the PGA 33, the ADC 34 and the digital signal processor 120. As soon as the signal processor 120 detects yarn(s) 2 within the monitoring volume 16, it sends a signal to the main controller 110 to stop the advance of the tying machine 100 relative to the associated warp sheet, by stopping the corresponding drive 101, 102, and the rotation of the associated spindle 301, by stopping the corresponding spindle drive 303.1, 303.2.

During insertion into the monitoring volume 16, each yarn 2 comes into contact with the back face 306 of the spindle 301, which constitutes an abutment surface 306, in the advance direction A and with the two support surfaces 55, 56 in the insert direction I. When in contact with the support surfaces 55, 56 and the abutment surface 306, a length portion of the yarn 2 between the two supports 53, 54 is in the monitoring volume 16. As the yarn inside the monitoring volume 16 is (still) clamped in the associated clamping device 111, 112 and deflected relative to plane P2.1, P2.2 of the associated warp sheet 2.1, 2.2, it rests against the support surfaces 55, 56 under tension. As the yarn 2 is also deflected in the advance direction A, the yarn 2 also rests against the abutment surface/ back face 306 of the spindle 301, at least at two portions of the yarn 2 spaced along the longitudinal direction L, these two portions being outside of the length portion of the yarn 2 which extends between the two support surfaces 55, 56. Due to the insertion under pretension load, the yarn 2 may bounce on the support surfaces 55, 56 and the abutment surface 306 and vibrate on both sides of the common plane 59. As the yarn does not move along the longitudinal direction, the capacitive sensor device 1 is only able to monitor a length portion of each yarn 2.

At least a second measurement may be done a short time after detection of the presence of a yarn in the monitoring volume 16 for monitoring the exact number of yarns in the monitoring volume 16. As the rotation of the spindle 301 and the advance movement are stopped, and as the yarn is supported by the two support surfaces, it allows for an accurate measurement with limited vibrations of the mechanical parts of the tying machine. The second analysis of the induced signal is done in order to determine the actual number of yarns in the monitoring volume 16 of the monitoring capacitor 11. A typical monitoring sequence may comprise the following steps:
- For the first yarn to be monitored during the separation process, the main controller 110 asks the operator to confirm the presence of a single yarn, e.g. via a human-machine interface such as a touchscreen 113 connected to the main controller 110. The respective monitoring signal constitutes an initial reference value which will be memorized. The single yarn is then taken out from the capacitive sensor device 1, e.g. by a transfer device, like a rotating clamping device, that brings the single yarn to a cutter and then the cut yarn end to the knotting unit of the tying machine 100 (not shown).
- For the next yarns to be measured, the procedure works as follows: If no yarn is detected by a second measurement, the signal processor 120 sends an absence signal to the main controller 110 to actuate again the rotation of the corresponding spindle 301 of the separating device 300 and its advance to finally separate a yarn 2 and stop advance and rotation of the corresponding spindle 301. If a single yarn is detected, the signal processor 120 sends a "normal" signal to the main controller 110 which actuates the corresponding transfer device to take out the single yarn 2 from the monitoring volume 16. The main controller 110 then actuates again the rotation and the advance of the corresponding spindle 301 of the separating device 300 to separate another yarn 2. Otherwise, if two or more yarns 2 are detected (higher induced signal), the respective signal processor 120 sends a "default" signal to the main controller 110 which rotates the corresponding spindle 301 back (e.g. by one turn) so that the released yarns 2 are brought back from the monitoring volume 16 into the helical groove 305 on the corresponding spindle 301, e.g. by a nose 307 at the back face 306 of the spindle 301 (see Fig. 2). Then the corresponding spindle 301 stops back-rotating and again rotates in the normal (transporting and separating) direction to separate again a yarn 2 at the releasing edge 304 of the corresponding spindle 301. The nose 307 is out of the yarn path when the yarn 2 falls from the spindle 301 into the monitoring volume 16. This process is repeated until a single yarn is detected.
- The separation process is continued on until all yarns 2 of the warp sheets 2.1 2.2 are separated.

In order to ensure that separation can only start if a single separated yarn has been correctly taken out from the capacitive sensor device 1 by the transfer device, the controller 110 compares the result of the capacitive sensor device 1 with information about the position of the drive 130.2 of the transfer device. As long as the capacitive sensor device 1 indicates that a yarn is still within the monitoring volume 16 while the transfer device is in a position in which a yarn is supposed to be taken out from the monitoring volume 16, the controller 110 does not activate the separation of further yarns.

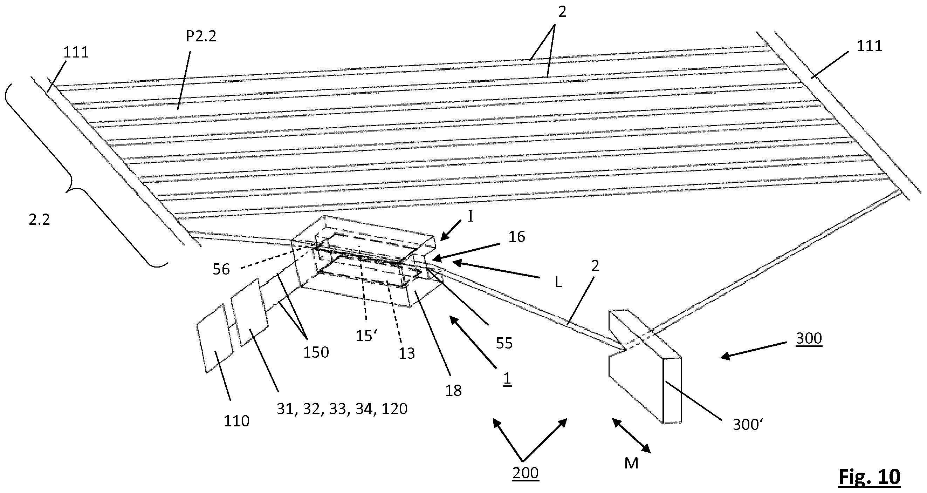

In a third embodiment of the yarn separating module 200 shown in Fig.10, the yarn separating device 300 comprises a suction nozzle 300' as described in EP 1 383 949 B1 which is moved to and fro relative to the warp sheet 2.2. The direction of movement M of the suction nozzle 300' is parallel to the warp sheet plane 2.2. When the suction nozzle 300' approaches the first yarn 2 at the edge of the warp sheet 2.2, the suction nozzle 300' subjects the first yarn 2 to a negative pressure that separates the yarn 2 from the warp sheet 2.2. The suction nozzle 300' is associated to a capacitive sensor device 1 which is very similar to the capacitive sensor device 1 according to the first and second embodiments. During backwards movement of the nozzle 300, away from the warp sheet 2.2, the separated yarn 2 is still suctioned and thereby is inserted into the monitoring capacitor 11 of the sensor device 1 along an insert direction I. The separated yarn 2 is brought in abutment with the two spaced support surfaces 55, 56 for monitoring the length portion of yarn 2 extending through the monitoring volume 16 between two support surfaces 55, 56 along the longitudinal direction L. As the yarn 2 is deflected and transferred into the monitoring volume 11 by the nozzle 300 within the warp sheet plane P2.2, the insert direction I is coplanar with the warp sheet plane P2.2. In Fig. 10, the yarn separating module 200 is only shown with a monitoring capacitor 11 having first and second electrodes 13, 15 (no compensation capacitor) and with support surfaces 55, 56 formed on an insulating member 18.

Of course, specific characteristics and features of the first, second and third embodiment may be combined.

With regard to the use in a warp drawing-in machine or in a warp tying machine, the separating device 300 separates yarns from leased warp sheets. The lease is open and the yarn(s) placed in the lease are transported to the monitoring volume of the monitoring capacitor. If the sensor detects a double yarn, the sensor sends a "default" signal to the main controller 110 which stops the warp tying machine and the main controller 110 asks, via a human-machine interface like the touchscreen 113, the operator to check the separation result. If two or more yarns extend in the monitoring volume, the operator shall remove the bad yarn (s) . Then the operator has to confirm the presence of a single yarn within the capacitive sensor 1 to the controller 110, via the touchscreen 113. After this, transfer of the single separated yarn out of the capacitive sensor and then separation of further yarns from the lease can occur.

In case of a tying machine 100, with one capacitive sensor 1 associated to each separating device 300 for both upper and lower warp sheets 2.1, 2.2, the main controller 110 of the tying machine 100 takes into account signals (separation result) from both signal processors 120 before actuating the knotting unit and supplying new separated yarns to the knotting unit.

In case of a yarn drawing-in machine which includes a separating module 200, having a single separating device 300 and one capacitive sensor 1 associated to a single warp sheet 2.1, the main controller 110 of the drawing-in machine 100 takes into account signals (separation result) from the signal processor 120 before actuating a gripper device that brings the separated yarn into harness elements of a weaving machine.

In case of a yarn drawing-in machine working with several warp sheets, for example two warp sheets 2.2, 2.1 (called "double beam"), which includes a separating module 200, having a separating device 300 and a capacitive sensor 1 associated to each warp sheet 2.1, 2.2, the main controller 110 of the drawing-in machine 100 takes into account signals (separation result) from the signal processor 120 of each capacitive sensor 1 before actuating the gripper device that brings the proper separated yarn into harness elements of the weaving machine.

In case of a leasing machine, with at least one separating module 200 comprising a separating device 300 and a capacitive sensor 1 for separating yarns from a single or several warp sheets, a main controller 110 of the leasing machine 100 takes into account signals (separation result) from the signal processor 120 associated to the yarn separating module 200 before actuating the leasing device that inserts the lease regarding to the separated yarn.

In another embodiment (not shown), the plane P15 of the common electrode 15 is perpendicular to the advance direction A. First and second electrodes 14, 15 of the compensation capacitor 12 are placed inside the spindle 301, whereas the first electrode 13 of the monitoring capacitor 11 is placed outside the spindle 301. The electrode plate 15 has an external curved edge which follows the curved circumferentially geometry of the spindle 301 and is inclined (10° to 25°, in particular 18°) relative to the first electrodes 13 and 14.

In another embodiment (not shown), the sender electrode is the plate electrode and the receiver electrode is the wire electrode.

In yet another embodiment (not shown), the separating device 300 comprises more than one spindle, for example two spindles each with helical groove and releasing edge, also described in EP 2 881 506. The capacitive sensor 1 and especially the monitoring volume 16 is placed at the back of the second (transport) spindle, opposite to the yarn sheet and to the first (separation) spindle, along the advance direction.

## Claims

1. A yarn separating module (200), comprising a yarn separating device (300) for separating a predetermined number of yarns (2), preferably a single yarn (2), from a yarn layer (2.1, 2.2), and a capacitive sensor device (1) for monitoring the yarn separation result, in particular the number of actually separated yarns (2), wherein the yarn separating device (300) is configured to transfer a length portion of the separated yarn(s) into a gap-like monitoring volume (16) of a monitoring capacitor (11) of the capacitive sensor device (1), the gap-like monitoring volume (16) being formed between a first electrode (13) and a second electrode (15, 15') of the monitoring capacitor (11) facing each other and spaced transverse to a longitudinal direction (L) of the length portion of the separated yarn(s) when extending through the monitoring volume (16) of the monitoring capacitor (11), wherein the capacitive sensor device (1) further comprises at least two support surfaces (55, 56) for supporting the separated yarn(s), the two support surfaces (55, 56) being spaced along the longitudinal direction (L) and fixed relative to the first and second electrodes (13, 15, 15'), the monitoring volume (16) of the monitoring capacitor (11) being arranged at least partially between the two support surfaces (55, 56).

2. The yarn separating module (200) according to claim 1, wherein the two support surfaces (55, 56) of the capacitive sensor device (1) define a common plane (59) intersecting the first and second electrodes (13, 15, 15') of the monitoring capacitor (11), each of the first and second electrodes (13, 15, 15') of the monitoring capacitor (11) extending on both sides of the common plane (59).

3. The yarn separating module (200) according to claim 2, wherein the common plane (59) intersects the first and the second electrodes (13, 15, 15') of the monitoring capacitor (11) at half height (h/2) with regard to a respective height extension (h) of the first and second electrodes (13, 15, 15') taken perpendicular to the common plane (59).

4. The yarn separating module (200) according to one of the preceding claims, wherein the yarn separating device (300) is configured to transfer a length portion of the separated yarn(s) (2) into the monitoring volume (16) of the monitoring capacitor (11) in an insert direction (I) and each support surface (55, 56) comprises a flat surface portion pointing opposite to the insert direction (I) and being perpendicular to the insert direction (I).

5. The yarn separating module (200) according to one of the preceding claims, wherein the first electrode (13) and the second electrode (15, 15') of the monitoring capacitor (11) respectively defines a first electrode plane (P13) and a second electrode plane (P15, P15'), and wherein each support surface (55, 56) extends perpendicularly to the first electrode plane (P13) and/or to the second electrode plane (P15, P15').

6. The yarn separating module (200) according to one of the preceding claims, wherein at least one of the first electrode (13) and the second electrode (15, 15') of the monitoring capacitor (11) is formed by a metallic coating on an insulating member (18).

7. The yarn separating module (200) according to one of the preceding claims, wherein each of the two support surfaces (55, 56) of the capacitive sensor device (1) is shifted from a plane (P2.1, P2.2) in which a yarn layer (2.1, 2.2) is to be arranged relative to the yarn separating device (300) for yarn separation.

8. The yarn separating module (200) according to one of the preceding claims, further comprising at least one abutment surface (306) for the separated yarn(s) to abut against when extending through the monitoring volume (16), wherein the abutment surface (306) defines an abutment plane (P306) intersecting and extending at an angle (α) between 90° and 105° with regard to the two support surfaces (55, 56) and wherein the abutment plane (P306) extends between the first (13) and second (15, 15') electrodes of the monitoring capacitor (11) and is spaced from the first and second electrodes of the monitoring capacitor (11) in a direction perpendicular to the abutment plane (P306).

9. The yarn separating module (200) according to one of the preceding claims, wherein the two support surfaces (55, 56) are formed by a casing (50) of the capacitive sensor device (1) in which an insulating member (18) holding the first and second electrodes (13, 15, 15') of the monitoring capacitor (11) is fixed, the two support surfaces (55, 56) delimiting the longitudinal sides of an insertion slot (52) of the casing (50) for inserting yarns (2) from the exterior of the casing into the monitoring volume (16) of the monitoring capacitor (11).

10. The yarn separating module (200) according to claim 9, further comprising an electronic circuit operatively connected to the first and second electrodes (13, 14), wherein at least parts of the electronic circuit include a programmable gain amplifier and are housed in the casing (50) of the capacitive sensor device (1).

11. The yarn separating module (200) according to one of the preceding claims, wherein the capacitive sensor device (1) further comprises a compensation capacitor (12), having a first electrode (14) and a second electrode (15, 15") forming a gap-like monitoring volume (17) therebetween, wherein all electrodes (13, 14, 15; 13, 14, 15', 15") of the capacitive sensor device (1) are aligned in a direction transverse to the longitudinal direction (L).

12. The yarn separating module (200) according to one of the preceding claims, wherein the separating device (300) comprises at least one rotating spindle (301) having an external helical groove (305), a back surface (306) and a releasing edge (304) at the intersection between the helical groove (305) and the back surface (306), wherein the capacitive sensor device (1) is placed at the back of the spindle (301) with one of the first and the second electrode (13, 15, 15') of the monitoring capacitor (11) placed inside the spindle (301).

13. A warp drawing-in machine comprising at least a yarn separating module (200) according to one of the claims 1 to 12, wherein a controller (110) of the drawing-in machine is operatively connected to the capacitive sensor device (1) of the at least one yarn separating module (200).

14. A warp tying machine (100) for joining yarns from two different yarn layers (2.1, 2.2), the warp tying machine (100) comprising a respective yarn separating module (200) according to one of claims 1 to 12 for each of the two yarn layers (2.1, 2.2), wherein a controller (120) of the warp tying machine (100) is operatively connected to the capacitive sensor device (1) of each yarn separating module (200) .

15. The warp tying machine (100) according to claim 14 further comprising a yarn transfer device associated to each yarn separating module (200) for taking out yarn(s) from the monitoring volume (16) of the sensor device, the controller (120) of the warp tying machine (100) being operatively connected to a drive (130.1, 130.2) of the yarn transfer device.

## Patentansprüche

1. Fadentrennmodul (200), umfassend eine Fadentrennvorrichtung (300) zum Trennen einer vorbestimmten Anzahl an Fäden (2), vorzugsweise eines einzelnen Fadens (2), von einer Fadenschicht (2.1, 2.2) und eine kapazitive Sensorvorrichtung (1) zum Überwachen des Fadentrennergebnisses, insbesondere der Anzahl an tatsächlich getrennten Fäden (2), wobei die Fadentrennvorrichtung (300) ausgelegt ist, einen Längenabschnitt des getrennten Fadens bzw. der getrennten Fäden in ein spaltartiges Überwachungsvolumen (16) eines Überwachungskondensators (11) der kapazitiven Sensorvorrichtung (1) zu überführen, wobei das spaltartige Überwachungsvolumen (16) zwischen einer ersten Elektrode (13) und einer zweiten Elektrode (15, 15') des Überwachungskondensators (11) ausgebildet ist, welche einander zugewandt und transversal zu einer Längsrichtung (L) des Längenabschnitts des getrennten Fadens bzw. der getrennten Fäden beabstandet sind, wenn sich der Längenabschnitt durch das Überwachungsvolumen (16) des Überwachungskondensators (11) erstreckt, wobei die kapazitive Sensorvorrichtung (1) ferner mindestens zwei Trageoberflächen (55, 56) zum Tragen des getrennten Fadens bzw. der getrennten Fäden umfasst, wobei die zwei Trageoberflächen (55, 56) entlang der Längsrichtung (L) beabstandet und bezüglich der ersten und der zweiten Elektrode (13, 15, 15') feststehend sind, wobei das Überwachungsvolumen (16) des Überwachungskondensators (11) mindestens teilweise zwischen den zwei Trageoberflächen (55, 56) angeordnet ist.

2. Fadentrennmodul (200) nach Anspruch 1, wobei die zwei Trageoberflächen (55, 56) der kapazitiven Sensorvorrichtung (1) eine gemeinsame Ebene (59) definieren, welche die erste und die zweite Elektrode (13, 15, 15') des Überwachungskondensators (11) kreuzt, wobei jeweils die erste und die zweite Elektrode (13, 15, 15') des Überwachungskondensators (11) an beiden Seiten der gemeinsamen Ebene (59) verlaufen.

3. Fadentrennmodul (200) nach Anspruch 2, wobei die gemeinsame Ebene (59) die erste und die zweite Elektrode (13, 15, 15') des Überwachungskondensators (11) auf halber Höhe (h/2) in Hinsicht auf eine jeweilige Höhenerstreckung (h) der ersten und der zweiten Elektrode (13, 15, 15'), senkrecht zur gemeinsamen Ebene (59) genommen, kreuzt.

4. Fadentrennmodul (200) nach einem der vorhergehenden Ansprüche, wobei die Fadentrennvorrichtung (300) ausgelegt ist, einen Längenabschnitt des getrennten Fadens bzw. der getrennten Fäden (2) in das Überwachungsvolumen (16) des Überwachungskondensators (11) in einer Einführrichtung (I) zu überführen, und jede Trageoberfläche (55, 56) einen flachen Oberflächenabschnitt umfasst, der in die entgegengesetzte Richtung der Einführrichtung (I) zeigt und senkrecht zur Einführrichtung (I) ist.

5. Fadentrennmodul (200) nach einem der vorhergehenden Ansprüche, wobei die erste Elektrode (13) und die zweite Elektrode (15, 15') des Überwachungskondensators (11) jeweils eine erste Elektrodenebene (P13) und eine zweite Elektrodenebene (P15, P15') definieren, und wobei jede Trageoberfläche (55, 56) senkrecht zur ersten Elektrodenebene (P13) und/oder zur zweiten Elektrodenebene (P15, P15') verläuft.

6. Fadentrennmodul (200) nach einem der vorhergehenden Ansprüche, wobei mindestens eine der ersten Elektrode (13) und der zweiten Elektrode (15, 15') des Überwachungskondensators (11) durch eine metallische Beschichtung auf einem Isolierelement (18) ausgebildet wird.

7. Fadentrennmodul (200) nach einem der vorhergehenden Ansprüche, wobei jede der zwei Trageoberflächen (55, 56) der kapazitiven Sensorvorrichtung (1) von einer Ebene (P2.1, P2.2) verschoben ist, auf der eine Fadenschicht (2.1, 2.2) bezüglich der Fadentrennvorrichtung (300) zur Fadentrennung angeordnet werden soll.

8. Fadentrennmodul (200) nach einem der vorhergehenden Ansprüche, ferner umfassend mindestens eine Anlageoberfläche (306), an welcher der getrennte Faden bzw. die getrennten Fäden anliegen kann bzw. können, wenn sich der getrennte Faden bzw. die getrennten Fäden durch das Überwachungsvolumen (16) erstreckt bzw. erstrecken, wobei die Anlageoberfläche (306) eine Anlageebene (P306) definiert, die bei einem Winkel (α) zwischen 90° und 105° bezüglich der zwei Trageoberflächen (55, 56) kreuzt und verläuft, und wobei die Anlageebene (P306) sich zwischen der ersten (13) und der zweiten (15, 15') Elektrode des Überwachungskondensators (11) erstreckt und von der ersten und der zweiten Elektrode des Überwachungskondensators (11) in einer zur Anlageebene (P306) senkrechten Richtung beabstandet ist.

9. Fadentrennmodul (200) nach einem der vorhergehenden Ansprüche, wobei die zwei Trageoberflächen (55, 56) durch ein Gehäuse (50) der kapazitiven Sensorvorrichtung (1) gebildet sind, in welcher ein Isolierelement (18) montiert ist, das die erste und die zweite Elektrode (13, 15, 15') des Überwachungskondensators (11) hält, wobei die zwei Trageoberflächen (55, 56) die Längsseiten eines Einführungsschlitzes (52) des Gehäuses (50) zum Einführen von Fäden (2) von der Außenseite des Gehäuses in das Überwachungsvolumen (16) des Überwachungskondensators (11) begrenzen.

10. Fadentrennmodul (200) nach Anspruch 9, ferner umfassend eine elektronische Schaltung, die mit der ersten und der zweiten Elektrode (13, 14) wirkverbunden ist, wobei mindestens Teile der elektronischen Schaltung einen programmierbaren Leistungsverstärker enthalten und im Gehäuse (50) der kapazitiven Sensorvorrichtung (1) untergebracht sind.

11. Fadentrennmodul (200) nach einem der vorhergehenden Ansprüche, wobei die kapazitive Sensorvorrichtung (1) ferner einen Kompensationskondensator (12) umfasst, der eine erste Elektrode (14) und eine zweite Elektrode (15, 15'') aufweist, die dazwischen ein spaltartiges Überwachungsvolumen (17) ausbilden, wobei alle Elektroden (13, 14, 15; 13, 14, 15', 15'') der kapazitiven Sensorvorrichtung (1) in einer zur Längsrichtung (L) transversalen Richtung ausgerichtet sind.

12. Fadentrennmodul (200) nach einem der vorhergehenden Ansprüche, wobei die Trennvorrichtung (300) mindestens eine rotierende Spindel (301) umfasst, die eine externe Spiralnut (305), eine hintere Oberfläche (306) und eine Freigabekante (304) am Kreuzungspunkt zwischen der Spiralnut (305) und der hinteren Oberfläche (306) aufweist, wobei die kapazitive Sensorvorrichtung (1) an der Rückseite der Spindel (301) mit der ersten oder der zweiten Elektrode (13, 15, 15') des Überwachungskondensators (11), der innerhalb der Spindel (301) angebracht ist, angebracht ist.

13. Kettfadeneinziehmaschine, umfassend mindestens ein Fadentrennmodul (200) nach einem der Ansprüche 1 bis 12, wobei eine Steuerung (110) der Einziehmaschine mit der kapazitiven Sensorvorrichtung (1) des mindestens einen Fadentrennmoduls (200) wirkverbunden ist.

14. Kettfadenknüpfmaschine (100) zum Verbinden von Fäden von zwei verschiedenen Fadenschichten (2.1, 2.2), wobei die Kettfadenknüpfmaschine (100) ein jeweiliges Fadentrennmodul (200) nach einem der Ansprüche 1 bis 12 für jede der zwei Fadenschichten (2.1, 2.2) umfasst, wobei eine Steuerung (120) der Kettfadenknüpfmaschine (100) mit der kapazitiven Sensorvorrichtung (1) jedes Fadentrennmoduls (200) wirkverbunden ist.

15. Kettfadenknüpfmaschine (100) nach Anspruch 14, ferner umfassend eine Fadenüberführungsvorrichtung, die jedem Fadentrennmodul (200) zugeordnet ist zum Herausnehmen eines Fadens bzw. von Fäden aus dem Überwachungsvolumen (16) der Sensorvorrichtung, wobei die Steuerung (120) der Kettfadenknüpfmaschine (100) mit einem Antrieb (130.1, 130.2) der Fadenüberführungsvorrichtung wirkverbunden ist.

## Revendications

1. Module de séparation de fils (200), comprenant un dispositif de séparation de fils (300) pour séparer un nombre prédéterminé de fils (2), de préférence un seul fil (2), d'une couche de fils (2.1, 2.2), et un dispositif de détection capacitif (1) pour contrôler le résultat de séparation de fils, en particulier le nombre de fils effectivement séparés (2), dans lequel le dispositif de séparation de fils (300) est configuré pour transférer une partie de longueur du/des fil(s) séparé(s) dans un volume de contrôle semblable à une fente (16) d'un condensateur de contrôle (11) du dispositif de détection capacitif (1), le volume de contrôle semblable à une fente (16) étant formé entre une première électrode (13) et une deuxième électrode (15, 15') du condensateur de contrôle (11) se faisant face l'une l'autre et espacées transversalement à une direction longitudinale (L) de la partie de longueur du/des fil (s) séparé(s) lors de l'extension à travers le volume de contrôle (16) du condensateur de contrôle (11), dans lequel le dispositif de détection capacitif (1) comprend en outre au moins deux surfaces de support (55, 56) pour supporter le(s) fil (s) séparé(s), les deux surfaces de support (55, 56) étant espacées le long de la direction longitudinale (L) et fixes par rapport aux première et deuxième électrodes (13, 15, 15'), le volume de contrôle (16) du condensateur de contrôle (11) étant disposé au moins en partie entre les deux surfaces de support (55, 56).

2. Module de séparation de fils (200) selon la revendication 1, dans lequel les deux surfaces de support (55, 56) du dispositif de détection capacitif (1) définissent un plan commun (59) coupant les première et deuxième électrodes (13, 15, 15') du condensateur de contrôle (11), chacune des première et deuxième électrodes (13, 15, 15') du condensateur de contrôle (11) s'étendant sur les deux côtés du plan commun (59).

3. Module de séparation de fils (200) selon la revendication 2, dans lequel le plan commun (59) coupe les première et deuxième électrodes (13, 15, 15') du condensateur de contrôle (11) à mi-hauteur (h/2) compte tenu d'une extension en hauteur (h) respective des première et deuxième électrodes (13, 15, 15') prise perpendiculairement au plan commun (59).

4. Module de séparation de fils (200) selon l'une des revendications précédentes, dans lequel le dispositif de séparation de fils (300) est configuré pour transférer une partie de longueur du/des fil(s) séparé(s) (2) dans le volume de contrôle (16) du condensateur de contrôle (11) dans une direction d'insertion (I), et chaque surface de support (55, 56) comprend une partie de surface plate pointant en opposition à la direction d'insertion (I) et étant perpendiculaire à la direction d'insertion (I).

5. Module de séparation de fils (200) selon l'une des revendications précédentes, dans lequel la première électrode (13) et la deuxième électrode (15, 15') du condensateur de contrôle (11) définissent respectivement un plan de première électrode (P13) et un plan de deuxième électrode (P15, P15'), et dans lequel chaque surface de support (55, 56) s'étend perpendiculairement au plan de première électrode (P13) et/ou au plan de deuxième électrode (P15, P15').

6. Module de séparation de fils (200) selon l'une des revendications précédentes, dans lequel au moins l'une parmi la première électrode (13) et la deuxième électrode (15, 15') du condensateur de contrôle (11) est formée par un revêtement métallique sur un élément isolant (18).

7. Module de séparation de fils (200) selon l'une des revendications précédentes, dans lequel chacune des deux surfaces de support (55, 56) du dispositif de détection capacitif (1) est décalée par rapport à un plan (P2.1, P2.2) dans lequel une couche de fils (2.1, 2.2) doit être disposée par rapport au dispositif de séparation de fils (300) pour la séparation de fils.

8. Module de séparation de fils (200) selon l'une des revendications précédentes, comprenant en outre au moins une surface de butée (306) pour le (s) fil (s) séparé(s) pour venir en butée lors de l'extension à travers le volume de contrôle (16), dans lequel la surface de butée (306) définit un plan de butée (P306) qui coupe et s'étend selon un angle (α) compris entre 90° et 105° compte tenu des deux surfaces de support (55, 56), et dans lequel le plan de butée (P306) s'étend entre les première (13) et deuxième (15, 15') électrodes du condensateur de contrôle (11) et est espacé des première et deuxième électrodes du condensateur de contrôle (11) dans une direction perpendiculaire au plan de butée (P306).

9. Module de séparation de fils (200) selon l'une des revendications précédentes, dans lequel les deux surfaces de support (55, 56) sont formées par un boîtier (50) du dispositif de détection capacitif (1) dans lequel un élément isolant (18) maintenant les première et deuxième électrodes (13, 15, 15') du condensateur de contrôle (11) est fixé, les deux surfaces de support (55, 56) délimitant les côtés longitudinaux d'une fente d'insertion (52) du boîtier (50) pour l'insertion de fils (2) à partir de l'extérieur du boîtier dans le volume de contrôle (16) du condensateur de contrôle (11).

10. Module de séparation de fils (200) selon la revendication 9, comprenant en outre un circuit électronique raccordé de manière fonctionnelle aux première et deuxième électrodes (13, 14), dans lequel au moins des parties du circuit électronique comprennent un amplificateur de gain programmable et sont logées dans le boîtier (50) du dispositif de détection capacitif (1).

11. Module de séparation de fils (200) selon l'une des revendications précédentes, dans lequel le dispositif de détection capacitif (1) comprend en outre un condensateur de compensation (12) ayant une première électrode (14) et une deuxième électrode (15, 15") formant un volume de contrôle semblable à une fente (17) entre elles, dans lequel toutes les électrodes (13, 14, 15 ; 14, 15', 15"") du dispositif de détection capacitif (1) sont alignées dans une direction transversale à la direction longitudinale (L).

12. Module de séparation de fils (200) selon l'une des revendications précédentes, dans lequel le dispositif de séparation (300) comprend au moins une broche rotative (301) ayant une rainure externe hélicoïdale (305), une surface arrière (306) et un bord de libération (304) à l'intersection entre la rainure hélicoïdale (305) et la surface arrière (306), dans lequel le dispositif de détection capacitif (1) est placé à l'arrière de la broche (301) avec l'une parmi la première et la deuxième électrode (13, 15, 15') du condensateur de contrôle (11) placée à l'intérieur de la broche (301).

13. Machine à rentrer des fils de chaîne comprenant au moins un module de séparation de fils (200) selon l'une des revendications 1 à 12, dans lequel un contrôleur (110) de la machine à rentrer est raccordé de manière fonctionnelle au dispositif de détection capacitif (1) de l'au moins un module de séparation de fils (200).

14. Machine de nouage de fils (100) pour joindre des fils de deux couches différentes de fils (2.1, 2.2), la machine de nouage de fils (100) comprenant un module de séparation de fils respectif (200) selon l'une des revendications 1 à 12 pour chacune des deux couches de fils (2.1, 2.2), dans lequel un contrôleur (120) de la machine de nouage de fils (100) est raccordé de manière fonctionnelle au dispositif de détection capacitif (1) de chaque module de séparation de fils (200).

15. Machine de nouage de fils (100) selon la revendication 14, comprenant en outre un dispositif de transfert de fils associé à chaque module de séparation de fils (200) pour sortir un/des fil(s) du volume de contrôle (16) du dispositif de détection, le contrôleur (120) de la machine de nouage de fils (100) étant raccordé de manière fonctionnelle à une commande (130.1, 130.2) du dispositif de transfert de fils.
